# EUROPEAN PATENT APPLICATION

(11) **EP 4 628 837 A2**
(43) Date of publication of application: **08.10.2025**
(21) Application number: 25190713.5
(22) Date of filing: 31.01.2021
(51) Int. Cl.: G01B 9/02001

(54) **APPARATUS AND METHOD FOR SPECTRAL DOMAIN OPTICAL IMAGING**

(30) Priority: 31.01.2020 AU 2020900264
(62) Divisional of application: 21746967.5
(71) Applicant: Alcon Inc., 1701 Fribourg (CH)
(72) Inventor: FRISKEN, Steven James, Vaucluse, 2030 (AU)
(74) Representative: Sonnenberg Harrison Partnerschaft mbB

(57) **Abstract**

Apparatus and methods are presented for spectral domain optical imaging, in particular for single shot 3-D spectral domain imaging of the retina of the human eye. In certain embodiments one or more 3-D images across elongated areas of an object are acquired, with scanning perpendicular to the long axis of the elongated areas for imaging extended volumes of the object. In preferred embodiments the captured light is sampled in the Fourier plane, in a dimension substantially perpendicular to the long axis, with a cylindrical lenslet array, while in other embodiments the captured light is sampled in the image plane. Apparatus and methods are also presented for hyperspectral imaging of the retina, with the illuminating beams preferably angled to suppress interference from corneal reflections. Apparatus and methods are also presented for multi-wavelength wavefront sensing, with simultaneous capture of light in two or more paths with different delays.

## Description

### Field of the Invention

The invention relates to apparatus and methods for spectral domain optical imaging, in particular for 3-D spectral domain imaging of the retina of the human eye. However it will be appreciated that the invention is not limited to this particular field of use.

### Related Applications

The present application claims priority from Australian Provisional Patent Application No 2020900264 filed on 31 January 2020, the contents of which are incorporated herein by reference.

### Background of the Invention

Any discussion of the prior art throughout the specification should in no way be considered as an admission that such prior art is widely known or forms part of the common general knowledge in the field.

Optical coherence tomography (OCT) is a widely used interferometric technique for studying biological samples including *in-vivo* tissue such as the human eye, with lateral and depth resolution, using information contained in the amplitude and phase of light reflected or scattered from the sample. Most current OCT systems utilise spectral domain techniques where the depth information is encoded in the spectral response of the interference signal, which can be recorded as a time-varying function of wavelength (swept source OCT) or by dispersing the interference signal and detecting the different wavelengths simultaneously along a detector array (spectrometer-based OCT).

Many OCT systems acquire depth-resolved data at discrete points, i.e. A-scans, and use beam scanning in one or two lateral dimensions to generate B-scans (depth-resolved data in one lateral dimension) or C-scans (depth-resolved data in two lateral dimensions). Faster acquisition is generally desirable irrespective of the type of scan, especially for reducing motion-induced artefacts with *in-vivo* samples. So-called 'snap shot' spectrometer-based OCT approaches, in which depth-resolved data from a slice or volume of an object is acquired in a single frame of a sensor array, are particularly advantageous for reducing motion-induced artefacts. This is in contrast to swept source OCT approaches relying on sweeping a wavelength and capturing multiple frames corresponding to each of the wavelengths, which are highly sensitive to submicron shifts of the object during acquisition. Published US patent application No 2014/0028974 A1 entitled 'Line-field holoscopy' discloses a single-shot B-scan technique in which cylindrical lenses produce a line illumination on an object and on a reference mirror, followed by dispersion of the combined return sample and reference beams along one axis of a two-dimensional (2-D) sensor array. Advantageously, the holoscopic nature of this technique, where the sensor array is imaged to the far field of the object, allows digital refocusing and aberration correction as well as a degree of confocal rejection. However for full 3-D (C-scan) imaging the illuminated line needs to be scanned in the orthogonal direction and the 2-D sensor array read out repeatedly, and with *in-vivo* samples it is generally difficult to maintain phase stability between the consecutive B-scans. Consequently the achievable resolution and depth of focus tends to be highly anisotropic.

As disclosed in published US patent application No 2016/0345820 A1 entitled 'High resolution 3-D spectral domain optical imaging apparatus and method', the contents of which are incorporated herein by reference, single-shot C-scan acquisition can be achieved if a 2-D area of an object is illuminated and the combined returning sample and reference wavefronts sampled in two lateral dimensions, e.g. with a 2-D lenslet array, and the resulting sampling points dispersed onto separate sets of pixels of a 2-D sensor array. Digital refocusing can then be applied to the tomograms. The lateral resolution depends largely on the NA of the objective lens and may for example be around 3 µm. A limitation with this technique is that the illuminated area on the object needs to be relatively small, of order 100 µm x 100 µm, to reduce the impact of multiple scattering and because of the limited number of sampling points offered by commercially available 2-D lenslet arrays. Tomograms of multiple adjacent or overlapping volumes can be acquired by moving the object relative to the illuminated area in one or two dimensions, then stitched together to image larger object volumes, with the overall acquisition speed limited by the frame rate of the 2-D sensor array.

Other disadvantages of the prior art include the presence of cross-talk signals creating artefacts or degradation of signal-to-noise ratio (SNR) induced by multiple scattering of light that is in part coherent with the signal being measured. Furthermore the acceptance NA of existing systems is relatively low, meaning only a small fraction of the scattered light is contributing to the signal so the power densities required for a given SNR need to be increased to compensate.

Hyperspectral imaging provides various contrast mechanisms for screening and assessment of a range of retinal pathologies by measuring reflected/scattered light or fluorescence spectra over a range of discrete wavelengths, typically 20 to 100 wavelengths over the range of the visible or near-IR. However existing hyperspectral imaging systems based on fundus cameras and illumination with various wavelengths have limitations in the interpretation of acquired spectral data because of the inability to discriminate the signal from background light that may arise from reflection from other surfaces, in particular from the cornea. Additionally, sequential acquisition of wavelengths provides challenges for alignment of the different frames, which can cause errors in the predicted spectra from each pixel. Furthermore conventional instruments for acquiring hyperspectral images of the retina require the pupil to be dilated, which increases the time required and is inconvenient for the patient.

Many of the important applications of OCT technology have been related to ocular imaging and in particular to imaging of the fundus over a wide field, which has proven to be a particular challenge due to the limited acquisition speed of scanning systems and their ability to register scans. This is especially apparent in OCT-A (Angiography) applications where the time variation in an OCT signal is used as an indicator of blood flow. OCT-A systems need to perform repeated measurements over a typical time interval of a few milliseconds at each required location on the retina. This generally implies slow and uncomfortable acquisitions when covering a very wide field of the retina, say > 75 degrees, often with compromised spatial resolution, as well as requiring extremely demanding registration of the scans to ensure that bulk motion of the eye is not misinterpreted as flow. Wide-field OCT-A images are hence prone to motion artifacts, often in the form of registration stripes.

OCT systems commonly utilise broadband sources such as superluminscent LEDs or rapidly swept lasers, i.e. sources with relatively low spectral coherence, for providing depth resolution in the interference signal. Although spatially incoherent sources can be used in full-field time domain OCT, time domain systems typically have reduced SNR compared to spectral domain OCT systems as well as additional noise when away from the focal plane, limiting their performance. Furthermore, typical commercially available OCT systems have been based on point scanning, utilising sources with high spatial coherence for capture of phase information. This poses quite low limits on the illumination levels which can be used in ocular imaging, because the light from such sources can be focused to small and therefore high intensity spots on the retina, consequently limiting the area that can be imaged with a good SNR.

Unless the context clearly requires otherwise, throughout the description and the claims the words 'comprising', 'comprises' and the like are to be construed in an inclusive sense as opposed to an exclusive or exhaustive sense. That is, they are to be construed in the sense of 'including, but not limited to'. Similarly, unless the context clearly requires otherwise, the word 'or' is to be construed in an inclusive sense rather than an exhaustive sense. That is, the expression 'A or B' is to be construed as meaning 'A, or B, or both A and B'.

### Object of the Invention

It is an object of the present invention to overcome or ameliorate at least one of the limitations of the prior art, or to provide a useful alternative. It is an object of the present invention in a preferred form to provide spectral domain OCT apparatus and methods for single shot acquisition of 3-D images of laterally elongated volumes of an object, and for imaging extended volumes of the object by scanning the illumination in one lateral dimension. It is another object of the present invention in a preferred form to provide apparatus and methods for *in-vivo* hyperspectral imaging of the retina with reduced interference from corneal reflections.

### Summary of the Invention

According to a first aspect of the present invention there is provided an apparatus for wavefront sensing comprising:
an illumination system comprising an optical source for illuminating, with a multi-wavelength optical beam, an area of an object;
an optical system for capturing an optical wavefront comprising light scattered or reflected from the illuminated area;
an element for splitting the captured light into two or more paths having different optical delays;
a spatial sampling element for simultaneously sampling, in one dimension, the captured
light in the two or more paths; and
a measurement system comprising a two-dimensional sensor array for simultaneous measurement, over a range of wavelengths, of the light in the two or more paths.

Preferably, the apparatus comprises a computer for analysing the measurement of the light in the two or more paths to provide a measure of the optical wavefront. In certain embodiments the apparatus is configured to illuminate the fovea of an eye and capture light reflected or scattered from the fovea, for providing a measure of the power or aberrations of the eye.

According to a second aspect of the present invention there is provided a hyperspectral imaging apparatus comprising:
an illuminator for producing a plurality of elongated beams having a wavelength range covering selected spectral reflection or absorption features of an object;
an optical system for projecting said plurality of elongated beams onto a corresponding plurality of areas on said object and for capturing light reflected, scattered or fluoresced from said areas;
a linear aperture array for confocal gating of the captured light reflected, scattered or fluoresced from said areas; and
a spectrometer comprising a two-dimensional sensor array for simultaneous detection, over a range of wavelengths, of the captured light reflected, scattered or fluoresced from said areas.

In certain embodiments the apparatus is configured for hyperspectral imaging of an object comprising the retina of an eye. The optical system is preferably configured to project the plurality of elongated beams towards the eye at angles selected to suppress the capture of specular reflections of the elongated beams from the cornea of the eye. In certain embodiments the optical system comprises a focal plane relay including an aperture for suppressing the capture of specular reflections of the elongated beams from the cornea of the eye. In certain embodiments the optical system comprises a dispersive element for angularly dispersing the plurality of elongated beams, so as to reduce the intensity of the elongated beams on the retina.

The apparatus may comprise a cylindrical lenslet array for focusing the captured light onto the apertures of the linear aperture array. In certain embodiments the apparatus comprises a birefringent element for polarisation-sensitive detection of the captured light.

Preferably, the apparatus comprises a beam splitter for directing elongated beams towards the object and for directing the captured light towards the spectrometer. In certain embodiments the beam splitter is configured to provide a reference beam for coherence-resolved detection of the captured light.

The apparatus may comprise a deflection element for moving the areas on the object in a direction substantially perpendicular to the long axes of the areas, for imaging larger areas of the object.

In certain embodiments the illuminator comprises: an optical source for emitting an elongated stripe of light; and an array of one-dimensional focusing elements for producing the plurality of elongated beams from the elongated stripe of light. The illuminator may comprise a beam redirection element for steering individual beams of the plurality of elongated beams in selected directions. The beam redirection element is preferably positioned at a focal plane of the array of one-dimensional focusing elements. Preferably, the optical source is substantially spatially incoherent. In certain embodiments the illuminator comprises an aperture for adjusting the spatial coherence of the light emitted from the optical source.

According to a third aspect of the present invention there is provided an apparatus for hyperspectral confocal imaging of a sub-surface region of an object with enhanced rejection of surface reflected light, said apparatus comprising:
an illuminator for forming a spatially structured illumination field for projection onto a sub-surface region of an object;
an optical power element positioned in the far field of the structured illumination field;
a spatially structured confocal aperture configured to accept light reflected, scattered or fluoresced from said sub-surface region of said object;
a spectrometer comprising a dispersive optical relay for projecting a wavelength dispersed image of the structured confocal aperture onto a two-dimensional sensor array; and
a spatially selective directional coupling element for directing at least a portion of the structured illumination field towards said object and for directing at least a portion of the reflected, scattered or fluoresced light towards said spatially structured confocal aperture, such that the capture of light reflected from a front surface of said object is suppressed.

In certain embodiments the apparatus is configured for hyperspectral confocal imaging of a sub-surface region of an object comprising the retina of an eye or the cornea of an eye.

According to an fourth aspect of the present invention there is provided a method for wavefront sensing, said method comprising the steps of:
illuminating, with a multi-wavelength optical beam, an area of an object;
capturing an optical wavefront comprising light scattered or reflected from the illuminated area;
splitting the captured light into two or more paths having different optical delays;
simultaneously sampling, in one dimension, the captured light in the two or more paths; and
simultaneously measuring over a range of wavelengths, with a measurement system comprising a two-dimensional sensor array, the light in the two or more paths.

According to a fifth aspect of the present invention there is provided a method for hyperspectral imaging, said method comprising the steps of:
producing a plurality of elongated beams having a wavelength range covering selected spectral reflection or absorption features of an object;
projecting said plurality of elongated beams onto a corresponding plurality of areas on said object;
capturing light reflected, scattered or fluoresced from said areas;
confocally gating the captured light reflected, scattered or fluoresced from said areas; and
simultaneously detecting over a range of wavelengths, with a spectrometer comprising a two-dimensional sensor array, the captured light reflected, scattered or fluoresced from said areas.

According to a sixth aspect of the present invention there is provided a method for hyperspectral confocal imaging of a sub-surface region of an object with enhanced rejection of surface reflected light, said method comprising the steps of:
forming a spatially structured illumination field;
projecting, with a system of optics comprising an optical power element positioned in the far field of the structured illumination field, light in said structured illumination field onto said sub-surface region of said object;
accepting, with a spatially structured confocal aperture, light reflected, scattered or fluoresced from said sub-surface region of said object;
projecting a wavelength dispersed image of the confocally accepted light onto a two-dimensional sensor array; and
directing, with a spatially selective directional coupling element, at least a portion of the structured illumination field towards said object and directing at least a portion of the reflected, scattered or fluoresced light towards said spatially structured confocal aperture, such that the capture of light reflected from a front surface of said object is suppressed.

### Brief Description of the Drawings

Preferred embodiments of the invention will now be described, by way of example only, with reference to the accompanying drawings in which:
Figure 1 illustrates in schematic oblique view an approach for mapping light reflected or scattered from an elongated illuminated volume of an object onto a 2-D sensor array;
Figure 2A shows in schematic plan view an optical imaging apparatus for *in-vivo* tomographic imaging of the retina of a human eye, according to an embodiment of the present invention;
Figure 2B depicts a portion of the apparatus shown in Figure 2A in an orthogonal plane;
Figure 3 shows in schematic oblique view an optical imaging apparatus according to an embodiment of the present invention;
Figure 4 illustrates in schematic plan view an optical imaging apparatus according to an embodiment of the present invention;
Figure 5 shows in schematic plan view an apparatus for multi-wavelength wavefront analysis of light reflected or scattered from an object, according to an embodiment of the present invention;
Figure 6A depicts in schematic plan view an apparatus for *in-vivo* wide field hyperspectral imaging of the retina of an eye, according to an embodiment of the present invention;
Figure 6B shows in schematic oblique view the construction of a light source suitable for use in the apparatus of Figure 6A;
Figure 6C shows in schematic plan view an alternative beam splitter for use in the apparatus of Figure 6A;
Fig 7A depicts in schematic plan view an apparatus for *in-vivo* wide field hyperspectral imaging of the retina of an eye, according to an embodiment of the present invention; and
Figure 7B depicts in schematic plan view an apparatus for hyperspectral confocal imaging of a sub-surface region of an object, according to an embodiment of the present invention.

### Detailed Description of the Invention

The current invention provides techniques and apparatus for snapshot tomographic imaging across an elongated volume of an object such as the retina of a human eye with resolution that is substantially isotropic in two or three dimensions, with application of digital refocusing in both lateral axes so that larger volumes of the object can be constructed with high resolution by shifting the elongated illuminated area substantially perpendicularly to its long axis. The full-field nature of the acquisition enables registration of phase between individual volume acquisitions. The technique is also suitable for high-resolution hyperspectral analysis or metrology over extended areas or volumes of an object. By selecting or controlling the spatial coherence of the illumination, multiply-scattered light can be suppressed while providing sufficient signal coherence for aberration correction and digital refocusing. In certain embodiments polarisation information can be extracted from an object by knowing or controlling the polarisation state of the illumination and utilising dual polarisation detection.

The cross-sectional area of an object that can be accessed in a single snapshot acquisition using the techniques of the present invention is considerably larger than in the prior art, and may be example be of order 3 mm x 50 µm compared to areas of order 100 µm x 100 µm in US 2016/0345820 A1. The ability to access larger areas in a single frame reduces the number of frames required to image a given overall area of an object, thereby reducing the total acquisition time. Furthermore with a highly elongated illumination area it may only be necessary to scan the illumination in one dimension rather than two, simplifying the apparatus and the registration of individual snapshot images. In basic terms the techniques of the present invention trade speed for imaging depth or number of resolvable wavelengths, allowing a larger area metric or resolution in a given acquisition time for a given 2-D sensor array. The techniques may be particularly advantageous for retinal imaging, where the features of interest typically occupy a depth range of only a few hundred microns compared to the ~10 mm depth range required for imaging of the anterior segment of the human eye. In particular, the reduction in the time required to image large areas of the retina will be beneficial for ameliorating the effect of motion artefacts in OCT Angiography.

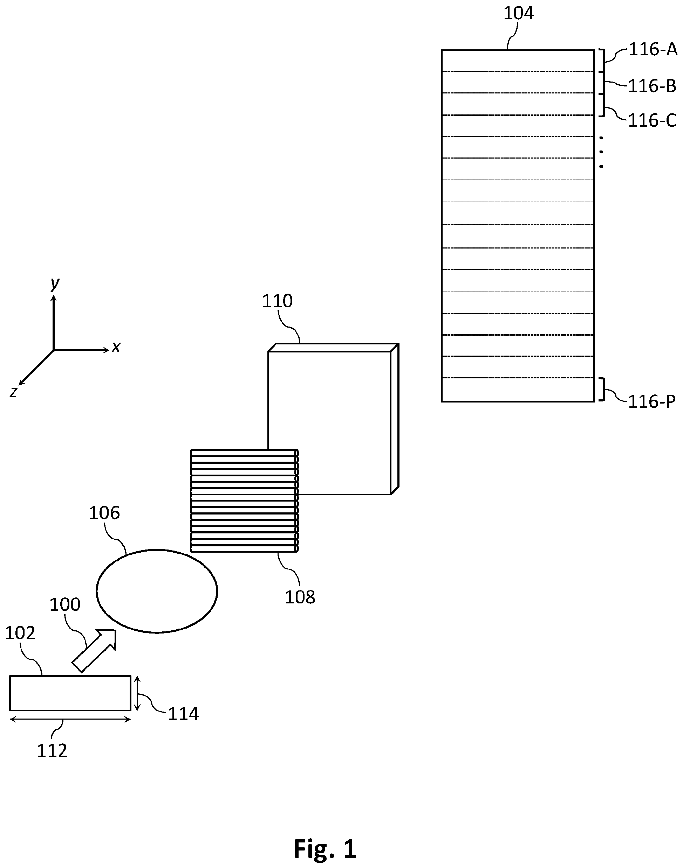

Figure 1 shows in schematic oblique view an approach for mapping light **100** reflected or scattered from an elongated illuminated volume of an object such as a retina, having a cross-sectional area **102,** onto a two-dimensional (2-D) sensor array **104,** using an optical system **106** illustrated for simplicity by a single lens, a spatial sampling element in the form of a cylindrical lenslet array **108** and a dispersive relay **110**. Spatial sampling may alternatively be provided by a linear aperture array or a combination of a lenslet array and an aperture array. As explained below, a suitable dispersive relay comprises a combination of lenses and a wavelength dispersive element such as a grating. The illuminated area **102,** shown here as rectangular although it may be any regular or irregular elongated shape, has an aspect ratio defined by the ratio of a long axis **112** to a short axis **114**. Depending on the details of the illumination source and optics, the aspect ratio of the illuminated area **102** may for example be at least ten, or at least twenty, or at least fifty, or at least one hundred.

In one specific example the illuminated area **102** is 9 mm x 48 µm, corresponding to an aspect ratio of 187.5, and the 2-D sensor array **104** is a 3000 x 4000 (12 Mpixel) array with 4 µm square pixels. It is relatively straightforward to image the long axis **112** of the illuminated area **102** onto the corresponding (x) axis of the sensor array **104,** with dispersion along the spectral (y) axis as in conventional line field OCT, to provide a lateral resolution of 3 µm in the x-axis (9 mm over 3000 pixels). However it is more challenging to obtain a snapshot C-scan with comparable resolution in the short axis **114** of the illuminated area **102.** In several embodiments described in detail below, a cylindrical lenslet array **108** samples the reflected or scattered light **100** in a dimension substantially parallel to the short axis **114,** with the optical system **106** configured to capture and anisotropically transform the reflected or scattered light **100.** In preferred embodiments the anisotropic transformation acts to reduce the aspect ratio of the captured light field such that its aspect ratio at the cylindrical lenslet array **108** is less than the aspect ratio of the elongated illuminated area **102.**

In certain embodiments the cylindrical lenslet array **108** is positioned at or near an image plane of the optical system **106,** for image plane sampling of the reflected or scattered light **100** in one dimension, or more precisely at multiple positions in one dimension, in this case the *y*-axis. The dispersive relay **110** then disperses the segmented image onto separate sections of the 2-D sensor array **104.** In the illustrated example a sixteen-element cylindrical lenslet array **108** samples the image in the *y*-axis for dispersion and projection onto separate sections **116-A, 116-B, 116-C...116-P** of the sensor array **104** as shown, effectively providing simultaneous acquisition of sixteen B-scans at the expense of a sixteen-fold reduction in depth range because of the reduced number of wavelengths per section **116** of the sensor array. However this image plane sampling approach requires the optical system **106** to have high magnification in the *y-*axis, which may be problematic in some applications, particularly where the cornea and intra-ocular lens of the eye contribute to the imaging of the reflected or scattered light **100.**

Surprisingly, we have found that high lateral resolution in the short axis **114** of the illuminated area **102** can be achieved for a spectral domain snapshot volume OCT imaging system, without requiring highly anisotropic image magnifying optics, by sampling the short axis **114** in the Fourier plane rather than in the image plane. In this case the cylindrical microlens array **108** is placed at or near a Fourier plane of the optical system **106,** converting lateral position along the short axis **114** to angle such that each section **116-A, 116-B...116-P** of the sensor array **104** receives information from across the short axis. That is, with Fourier plane sampling there is no 1:1 correspondence between the sections **116-A, 116-B...116-P** of the sensor array **104** and segments of the reflected or scattered light **100,** unlike the case with image plane sampling.

Irrespective of whether the *y*-axis sampling is in the Fourier plane or the image plane, sampling of the reflected or scattered light **100** in the *x*-axis is provided by the pixels of the sensor array **104.** This x-axis sampling may be in the Fourier plane or the image plane, depending among other things on the configuration of the optical system **106.** For efficient use of the 2-D sensor array **104** the optical system **106** and cylindrical lenslet array **108** are preferably configured such that the reflected or scattered light **100** is projected onto a substantial portion of the 2-D sensor array, generally with some margin for relaxed alignment tolerance. High-speed 2-D sensors with a large pixel count, e.g. multi-Mpixel sensors, enable capture of a large amount of data in a single frame, either with a global shutter or a rolling shutter. In situations where acquisition speed is paramount it is often desirable to restrict the field of the sensor to achieve faster frame rates. The present application on the other hand takes advantage of simultaneous acquisition, so to maximise the amount of data captured in a single frame it is preferable to utilise a substantial portion of the 2-D sensor array, i.e. to have a high pixel count in both dimensions. In state of the art visible or near IR light sensors this could mean projection onto at least 2000 pixels per dimension, but in any case it is expected that for various types of 2-D sensors more than 100 pixels per dimension would be illuminated.

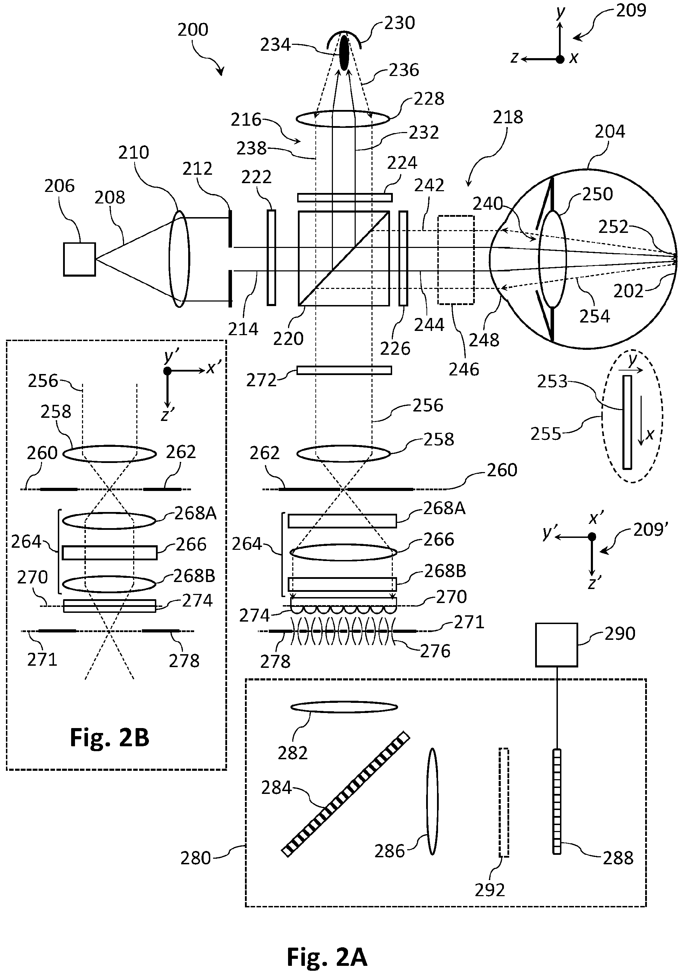

Figure 2A shows in schematic plan view an optical imaging apparatus **200** for *in-vivo* tomographic imaging of the retina **202** of a human eye **204,** according to an embodiment of the present invention. The apparatus **200** comprises a broadband line source **206** emitting a multi-wavelength beam of light **208** elongated in the x-axis, i.e. into the page, as defined by the Cartesian axes **209.** In certain embodiments the line source **206** is a spatially coherent source, e.g. a single spatial mode source such as an optical fibre-based supercontinuum source or a superluminescent light emitting diode (SLED) with appropriate anamorphic or astigmatic lensing. Preferably however the source **206** is a partially spatially incoherent source such as a high-power semiconductor gain medium having a stripe configuration, where feedback has been suppressed by antireflection coatings or angled facets allowing the gain medium to be driven below lasing threshold to produce broadband or partially incoherent light. The use of low coherence sources is generally advantageous for safety in *in-vivo* ocular examination because the emitted light cannot be focused to a small high-intensity spot on the retina. Other suitable partially spatially incoherent line sources include linear arrays of SLEDs, LEDs or wavelength-broadened VCSELs or laser diodes. In some embodiments a partially spatially incoherent source is provided by scanning one or more spatially coherent sources, such as an array of SLEDs, along the x-axis to create an elongated source. Within a single acquisition frame of a 2-D sensor array **288** the temporal incoherence between the different source positions achieves a degree of virtual spatial incoherence. Advantageously, this source scanning scheme allows a longer exposure time without fringe washout, and hence the use of lower peak power sources for the same average power.

In yet other embodiments the line source **206** is fully spatially incoherent, derived for example from laser-driven plasma, fluorescent or phosphorescent sources that may be confined within a single mode planar waveguide and pumped by another light source. When the object being imaged is an eye **204** as shown, the apparatus **200** will typically have a source **206** emitting light in the visible or near infrared regions of the electromagnetic spectrum. However this is not a limitation of the present invention and in general light sources emitting in the ultraviolet, visible or infrared regions of the electromagnetic spectrum may be used depending on the application. An etalon may be included to extend the coherence length of the light emitted from the line source **206.**

The elongated beam **208** emitted from the source **206** is collimated by a spherical lens **210** with focal length of, say, 20 mm, then passed through an optional aperture **212** that restricts the numerical aperture (NA) of the lens **210** and hence the minimum diffraction limited spot size that can be created if the apertured beam **214** is subsequently focused with a lens of a given focal length. The size of the aperture **212** can also be chosen to adjust the spatial coherence of the beam **208** emitted from the source **206,** with a smaller aperture generally increasing the spatial coherence of the transmitted light **214.** In certain embodiments the aperture **212** is circular, while in other embodiments the aperture is rectangular to restrict the beam **214** to different extents in the *x*- and *y*-axes.

The apertured source beam **214** is split into a reference path **216** and a sample path **218** with a beam splitter **220.** In the illustrated embodiment the beam splitter **220** is a polarisation beam splitting cube that splits the apertured source beam **214** according to the relative angle of the beam's polarisation state, which may be set by a polarising element **222.** A power beam splitter may be used instead, although the combination of a polarisation beam splitter **220** and quarter wave plates **224, 226** is generally less wasteful of source light.

The reference path **216** comprises an optical power element **228** in the form of a lens and a curved reflective element **230.** The optical power element **228** focuses the collimated beam **232** to a waist **234** that may for example be 100 µm by 20 mm. The curved reflective element **230** may be a short focal length cylindrical mirror that transforms the beam waist **234** in the short axis to be an angularly divergent beam **236.** This divergent beam is collimated by the lens **228** to form a reference beam **238** of comparable dimension to a dilated pupil **240,** suitable for mixing with the returning sample beam **242** as described below. For applications where shaping of the reference beam **238** is not required, the lens **228** and curved reflective element **230** may be replaced by a planar mirror.

Multi-wavelength light **244** in the sample path **218** is directed to the eye **204,** preferably by a 4F lens relay system **246** which may have focusing and angular beam steering capabilities as described for example in published US patent application No US 2019/0223729 A1 entitled 'Apparatus and method for confocal microscopy using dispersed structured illumination', the contents of which are incorporated herein by reference. The combined focusing power of the cornea **248** and intra-ocular lens **250** transforms the multi-wavelength sample beam **244** to a laterally elongated beam waist that illuminates a volume **252** of the retina **202,** with an elongated cross-sectional area **253** as shown in the inset view **255.** The illuminated area **253** on the retina, i.e. the beam waist dimensions, will generally be of order tens of microns by several millimetres in the y- and x-directions respectively, for example 50 µm x 3 mm corresponding to an aspect ratio of sixty. The illuminated area **253** may be substantially rectangular or elliptical in shape, or any other elongated shape with a longer dimension significantly larger than a shorter dimension. In preferred embodiments with a partially spatially incoherent line source **206** the light at the beam waist is relatively coherent across the shorter dimension, but coherent across only a small distance, e.g. 50 µm, in the longer dimension. On the other hand if the light is derived from a single spatial mode source **206** the projection onto the retina **202** will be coherent in both axes but will still have a highly elongated shape, e.g. 50 µm x 3 mm. Noting again the influence of the aperture **212** on the spatial coherence of the sample beam **244,** it is preferable for there to be some coherence across the long axis of the illuminated area **253** to allow digital refocusing.

Light **254** will be reflected or scattered from the illuminated volume **252** of the retina **202** in a large range of angles and can be captured within an angular range determined by the size of the dilated pupil **240,** with the larger numerical aperture components enabling higher resolution imaging of the retina **202.** The reflected or scattered light **254** will be collimated, at least for the case of an emmetropic eye, by the intra-ocular lens **250** and cornea **248** to form a returning sample beam **242.** A polarisation component of this returning sample beam is reflected by the polarisation beam splitter **220,** forming a return beam **256** with the substantially overlapping reference beam **238.** At this point the returning sample beam **242** and reference beam are orthogonally polarised so will not interfere until they are analysed by a polariser **272.** Since the reflectivity of the retina **202** will generally be significantly less than that of the reference arm mirror **230,** it is usually advantageous for the polariser **272** to be oriented to pass a large fraction of the returning sample beam **242** and a smaller fraction of the reference beam **238.** If on the other hand the sample and reference paths **218, 216** are split and recombined with a power beam splitter the returning sample beam **242** and reference beam **238** will interfere as soon as they are recombined.

A spherical lens **258** images the illuminated area **253** of the retina **202** to an image plane **260** where there is positioned a linear, i.e. slit-shaped, aperture **262** for excluding light which has been multiply-scattered or is from the edges of the illuminated volume **252.**

Light passing through the aperture **262** proceeds to an anisotropic relay system **264** of cylindrical lenses, the operation of which is described with reference to a set of Cartesian axes **209'** rotated with respect to the Cartesian axes **209** to account for the redirection at the beam splitter **220.** For ease of explanation the relay system **264** and surrounding optics are also illustrated in the orthogonal (*x'z*') plane in Figure 2B. The relay system **264** comprises a first cylindrical lens **266** of focal length 2*f* for focusing in a first axis (the *y*'-axis) and a pair of cylindrical lenses **268A, 268B** of focal length *f* for focusing in a second axis (the *x*'-axis). The first cylindrical lens **266** is positioned at a distance from the aperture **262** equal to its focal length 2*f* and acts to collimate the return beam **256** in the first axis, i.e. the short axis of the aperture **262.** The pair of cylindrical lenses **268A, 268B** act to collimate then refocus the light from the aperture **262** in the second axis, i.e. the long axis of the aperture **262.** The first cylindrical lens **266** therefore forms a 2F relay system in the first axis while the cylindrical lenses **268A, 268B** form a 4F relay system in the second axis, such that the plane **270** is a Fourier plane for light in the first axis and the plane **271** is an image plane for light in the second axis. The plane **271** is preferably displaced from the plane **270** by an optical equivalent distance determined by approximately twice the focal length of the lenslets in a cylindrical lenslet array **274** placed at or near the plane **270.**

The cylindrical lenslet array **274** forms a spatial sampling element for sampling the return beam **256** at multiple positions in the first (*y'*) axis, i.e. the axis for which the plane **270** is a Fourier plane, effectively sampling the return beam from different parts of the far field. The resulting array of focused lines **276** pass through an array of linear apertures **278** placed at or near the plane **271** before proceeding to a measurement system **280** for simultaneous measurement of phase and amplitude information over a range of wavelengths of the captured return beam **256** sampled by the cylindrical lenslet array **274.** By selecting or controlling the spatial coherence of the illumination, multiply-scattered light can be suppressed while providing sufficient signal coherence for aberration correction and digital refocusing. This scheme is advantageous in that it is able to physically separate the wavefront measurements of numerous sample regions that are each illuminated by light that is not spatially coherent to generate a full volume SD-OCT image while avoiding the noise penalties and sensitivity loss that would occur if the incoherent signals were captured overlapping on the same set of pixels. The measurement system **280** comprises a collimating lens **282,** a dispersive element **284** and a focusing lens **286** that creates dispersed line arrays for detection at a 2-D sensor array **288.** In the illustrated embodiment the dispersive element **284** is a transmissive grating, but in other embodiments it may for example be a prism, a reflective grating or a combination of a prism and a grating.

The relay system **264** of cylindrical lenses and the spherical lens **258** form an optical system configured to cooperate, when in use, with the optical power elements **248, 250** of the eye **204** for capturing and anisotropically transforming light **254** reflected or scattered from the illuminated volume **252** of the retina **202.** It will be appreciated that this optical system could be reconfigured for imaging other structures in the eye **204,** such as the intraocular lens **250** or the cornea **248.** Preferably, the anisotropic transformation serves to increase the spatial extent of the captured light in the first axis, corresponding to the short axis of the illuminated area **253,** relative to the second axis, so that the aspect ratio of the captured light at the cylindrical lenslet array **274** is less than the aspect ratio of the illuminated area **253.** In the illustrated embodiment the spatial extent of the captured light in the first (short) axis is determined by appropriate choices of the focal lengths of the spherical lens **258** and cylindrical lens **266.** In the illustrated example the far field at the dilated pupil **240** is expanded by a factor of two onto the cylindrical lenslet array **274,** e.g. from 8 mm to 16 mm, by choosing the focal length of the spherical lens **258** to be half that of the cylindrical lens **266.** It should be noted however that in this embodiment an anisotropic transformation of the reflected or scattered light is ensured by the fact that the reflected or scattered light is sampled in the Fourier plane in one axis and in the image plane in a second axis, by the cylindrical lenslet array **274** and the pixels of the 2-D sensor array **288** respectively.

In one particular embodiment the 2-D sensor array **288** is a high-speed Flash 4K 4000x1000 pixel sensor from Teledyne Technologies, Inc, capable of operating at 1800 frames per second (fps). With a 16-element cylindrical lenslet array **274** sampling the return beam **256** in the Fourier plane, and with 200 wavelengths per sample for dispersion along the 4000-pixel axis of the sensor array **288,** the apparatus **200** is able to provide the equivalent of 16x1000x1800 A-scans per second. This equates to 28.8M A-scans per second, which is approximately 100 times faster than the fastest currently available spectral domain OCT systems, which offer around 250k to 300k A-scans/sec. In another embodiment the 2-D sensor array **288** is a 12Mpixel (4000x3000) sensor providing 3000x16 = 48k A-scan equivalent resolution per frame, so that even a moderately low speed sensor, with a frame rate of say 60 Hz, could provide nearly 3M A-scans per second, enabling low-cost but high-speed retinal imaging.

Phase and amplitude data acquired with the 2-D sensor array **288** are processed with a computer **290** equipped with suitable machine-readable code to recover the retinal image at high resolution over a range of depths, e.g. with an on-retina pixel size of approximately 3x3 microns. Even over a small range of axial depths the line scan images will often become defocused along their length, necessitating some form of corrective processing such as digital refocusing.

Noting that the light **254** reflected or scattered from the retina **202** has been sampled in the Fourier plane in a first axis and in the image plane in a second axis, by the cylindrical lenslet array **274** and the pixels of the 2-D sensor array **288** respectively, one approach to processing the data will be described with reference to Figure 1.

In a first step, phase and amplitude data from each of the sections **116-A...116-P** of the 2-D sensor array **104,** which may for example have 200 resolvable wavelengths in the dispersive (y) axis, are Fourier transformed in the dispersive axis to transform from wavelength to axial depth, then Fourier transformed in the x-axis so that the data in both axes is now in the Fourier domain, i.e. the optical far field. We now have the complete volume 3-D Fourier information. In a second step the data is convolved with a focusing or aberration function, which may be termed a 'digital adaptive optics function', or 'DAO function', that can be determined iteratively for each depth to be visualised. In certain embodiments the choice of focusing or aberration function is determined analytically or iteratively by using an image focus metric or 'guide star' as may be provided by the reflections off individual cones in the retina. Finally, for each depth and choice of DAO function the 3-D data set is Fourier transformed to recreate the desired focal plane as is well known in the art. In general the resulting image will be a representation of an optical characteristic of the retina, such as phase, reflectivity, refractive index, refractive index changes and attenuation over the illuminated volume **252.** If the apparatus **200** is configured for polarisation diverse detection, e.g. with the addition of a polarisation separation element such as a walk-off plate **292** between the focusing lens **286** and the 2-D sensor array **288,** the optical characteristic may be birefringence or degree of polarisation.

To image larger regions of the retina **202,** beam steering optics in the lens relay system **246** can move the illuminated volume **252** to one or more adjacent or nearby locations, preferably with some overlap for co-registration of acquired data, and preferably in a direction substantially perpendicular to the long axis of the illuminated area **253,** i.e. in a direction substantially parallel to the y-axis. A tomographic image of an extended volume of the retina **202** can thereby be constructed from the series of snapshot acquisitions in an approach similar to the line field holoscopy technique described in published US patent application No 2014/0028974 A1, but with the significant advantage that high resolution is maintained in both lateral axes. Consequently the full volume will have high resolution in both axes even in the presence of lateral or axial shifts between adjacent snapshot acquisitions. Preferably the apparatus **200** is configured so that the resolution is substantially isotropic in both lateral axes.

For OCT-A applications the apparatus **200** can be configured for capture of phase and amplitude information in two or more sequential frames of the sensor array **288** at a given location on the retina **202,** optionally with pulsing of the source **206,** for processing in the computer **290** to allow measurement of changes due to blood flow in the illuminated volume **252.** A frame rate of 300 Hz for example would provide a 3.33 ms delay between frames, suitable for robust measurement of changes due to blood flow, whereas the eye motion will be small enough for there to be a significant overlap volume between the sequence of frames to allow co-registration and subtraction of the bulk eye motion. The process can be repeated at additional locations on the retina **202,** preferably with partial overlap for registration, for measuring blood flow in larger portions of the retina. In other embodiments the motion in the y- axis of the illumination between each frame can be small compared to the y-dimension of the illuminating beam such that an overlap region of the reconstructed volume can be used for determining the blood flow in a volume or projection image of the retina for example.

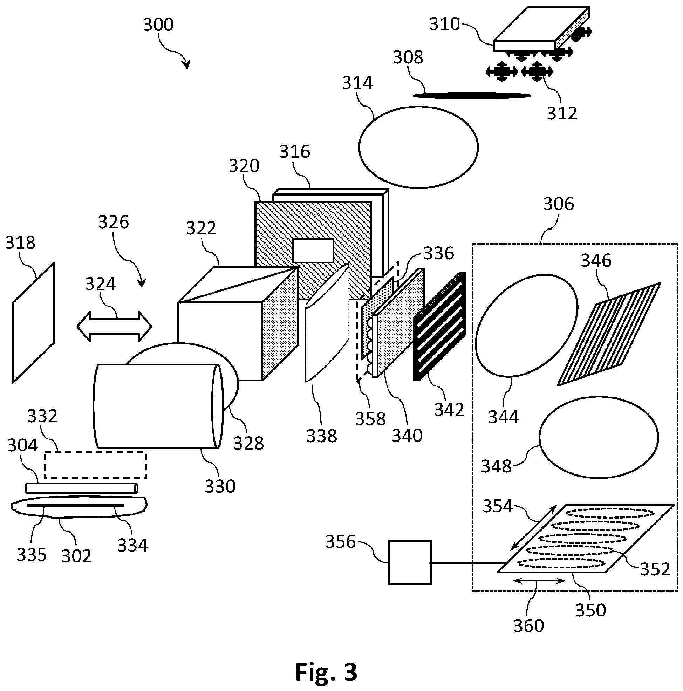

Figure 3 shows in schematic oblique view an optical imaging apparatus **300** for imaging an object **302,** which may for example be the cornea of an eye or a non-ocular biological sample, according to another embodiment of the present invention. In this embodiment an anisotropic magnification of the focal plane of a cylindrical objective lens **304** onto a 2-D spectrometer **306** enables a cylindrical lenslet-based optical system to image a contiguous volume of the object **302.** A highly asymmetric beam **308** of spatially incoherent and partially spectrally incoherent light is generated from a thin plate **310** of a phosphor crystal such as Ce-YAG pumped by an array of blue LEDs **312.** Incoherent light from the phosphor ions is collimated by an optical power element **314,** shown here as a lens but which could equally be a concave mirror, and the resultant beam passed through an optional Fabry-Perot etalon **316** to extend the coherence length of the individual spectral components. In one particular example a Fabry-Perot etalon **316** with a free spectral range of 200 GHz and a finesse of 20 may be chosen to yield a bandwidth of approximately 10 GHz in each spectral bin to provide improved tolerance for length matching the coherent interference provided by a reference mirror **318.** An aperture **320** may be included to provide a desired level of spatial coherence at the object **302,** with a smaller aperture providing greater spatial coherence, and hence range of digital refocusing, at the expense of reduced intensity.

A portion of the apertured light is reflected by a beam splitter **322** to form a reference beam **324,** which is reflected by a mirror **318.** This embodiment utilises a power beam splitter **322,** although a polarisation beam splitter could be used instead. Additional elements or features may be included in the reference arm **326,** e.g. shaping of the reference mirror **318** to ensure optimal overlap with the returning sample light when being redirected towards the spectrometer **306.** Source light that passes undeflected through the beam splitter **322** is directed to a combination of a spherical lens **328** and a relatively weak cylindrical lens **330** to provide focusing in one axis at a focal plane **332** of a short focal length cylindrical lens **304** such as a rod lens, illuminating a volume **334** of the object **302** having a highly elongated cross-sectional area **335.** In one particular example the combination of a spherical lens **328,** cylindrical lens **330** and rod lens **304** with respective focal lengths of 50 mm, 500 mm and 2 mm produces an illuminated area **335** of 200 µm x 12 mm on the object **302,** corresponding to an aspect ratio of sixty.

Light reflected or scattered from the illuminated volume **334** of the object **302** is captured and anisotropically transformed with an optical system comprising a cylindrical lens **338** in addition to the lenses **304, 330** and **328,** and relayed via the beam splitter **322** where it mixes with the reference beam **324.** In this embodiment this optical system magnifies the captured light to different extents in the short and long axes of the illuminated area **335**, with the cylindrical lens **338** and the spherical lens **328** providing magnification in the long axis of the illuminated area **335** and the magnification in the orthogonal short axis provided by the ratio of the focal length of the combined lens **328, 330** to the focal length of the cylindrical objective lens **304.** In one particular example a 25x magnification can be provided by a focal length ratio of 50 mm to 2 mm, enabling a 200 µm wide short axis to be magnified to a 5 mm wide image **336** that can be sampled with a spatial sampling element in the form of a cylindrical lenslet array **340** and a linear aperture array **342.** Preferably the apparatus is configured such that the lenslet array **340** is at or near the short axis image plane **358,** while the aperture array **342** is at or near the long axis image plane.

Light passed by the aperture array **342** proceeds to a 2-D spectrometer **306** comprising a collimating lens **344**, a grating **346,** a focusing lens **348** and a 2-D sensor array **350,** for simultaneous measurement of phase and amplitude information over a range of wavelengths. Portions of the short axis image **336** sampled by the cylindrical lenslet array **340** are dispersed onto separate portions **352** of the 2-D sensor array **350** separated in the dispersive axis **354,** with columns of pixels in the orthogonal direction **360** providing lateral resolution in the long axis of the illuminated area **335**. In one particular example, with a 12Mpixel (4000x3000) sensor array **350,** the magnified image **336** of the 200 µm wide short axis is sampled with a forty-element cylindrical microlens array **340** to provide a lateral resolution of 5 µm in that axis, while the 12 mm long axis is projected across 3000 pixels of the 2-D sensor array **350** to provide a lateral resolution of 4 µm in the long axis of the illuminated area **335.**

Phase and amplitude data acquired with the 2-D sensor array **350** can then be processed with a computer **356** equipped with suitable machine-readable code to recover depth-resolved information with lateral resolution in two axes across the illuminated volume **334.** The processing differs from that described for the apparatus **200** of Figure 2A because the holoscopy information is now in the image plane in both axes, with lines from the cylindrical lenslets **340** corresponding to spatial pixels in the short axis of the illuminated area **335.** In one approach the data set read out by the computer **356** is Fourier transformed and relevant aberration or focus corrections applied to provide a high-resolution image throughout the whole illuminated volume **334.** This scheme is likely to be advantageous for objects where the imaging depth requirement, and hence the number of wavelengths required, is quite limited, so the anisotropic imaging ratio is not too high for a practical optical design without overly large focal length requirements, advantageous for a compact apparatus.

The apparatus **300** may be advantageously applied to scanning applications where the apparatus is translated relative to the object **302** by moving the apparatus or the object. For example handheld instruments for scanning of skin for assessing burns or skin conditions such as melanoma can benefit hugely from the high-speed acquisition of volume information with post-processing of the focusing. Because of the reduced number of wavelengths that need to be captured per sampling point, the apparatus can be sufficiently compact for handheld use. It is in fact the digital refocusing that provides the opportunity to use a smaller number of wavelengths, as the apparatus does not rely solely on the coherence gate to provide depth resolution. Instead, the coherence gate is used in combination with digital refocusing to extend the range of imaging depth beyond the Nyquist range that would otherwise be offered by a limited set of wavelengths, with the extended depth range related to the extended coherence length provided by the etalon **316.** We note that the wavelength filtering provided by the etalon will generally be non-uniform across the illuminated volume **334** because of the range of emission angles from the phosphor ions in the light source **310,** however this can be calibrated across the beam to ensure accurate scaling of the axial depth.

In an alternative embodiment the cylindrical lens **338** is omitted, in which case the pixels of the sensor array **350** will sample the reflected or scattered light in the far field rather than the near field. This situation, where the sampling is in the near field (image plane sampling) in the short axis and in the far field (Fourier plane sampling) in the orthogonal long axis, is similar to the situation depicted in Figure 2A, except the axis of focusing and the Fourier plane have been swapped relative to the dispersive axis **354.**

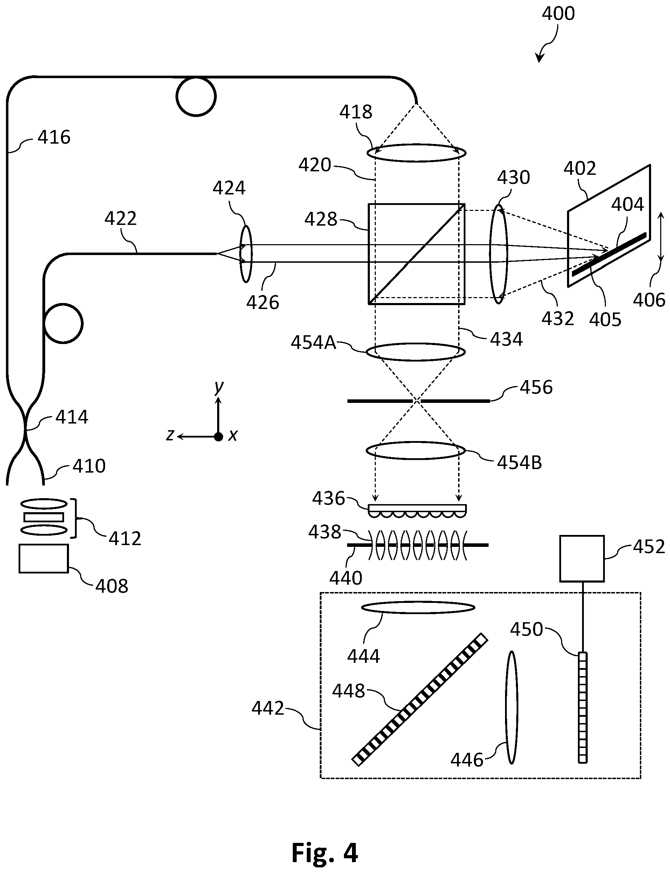

Figure 4 shows in schematic plan view an optical imaging apparatus **400** according to another embodiment of the present invention, in which relatively large areas or volumes of an object **402** such as a cornea or a non-ocular biological sample can be imaged by scanning an elongated illuminated area **405** in a direction **406** substantially parallel to the short axis of the area **405.** The scanning may for example be effected by lateral translation of the object or by angular scanning of a beam-steering mirror. In this embodiment a cylindrical lenslet array **436** provides far field sampling of reflected or scattered light **432** in a direction substantially parallel to the short axis of the illuminated area **405,** with dense far field sampling in a direction substantially parallel to the long axis provided by the pixels of a 2-D sensor array **450.** In preferred embodiments the aspect ratio of the illuminated area **405** is at least 10:1. The ability to image relatively large volumes of an object **402** with scanning in only one axis, rather than in two axes as in the substantially symmetrical sampling techniques disclosed in the above-mentioned US 2016/0345820 A1, simplifies the optics, which is advantageous for low cost and handheld applications.

The apparatus **400** utilises a spatially coherent but spectrally incoherent light source **408** such as a supercontinuum source coupled to an optical fibre **410.** A fibre-coupled source is advantageous for handheld applications because it can be located remotely from the remainder of the instrument. In the illustrated embodiment a fibre-coupled etalon filter **412** is included to pass a comb of wavelengths within a wavelength range of the light source **408,** so as to extend the coherence length for each of the wavelengths in the comb. A fraction of the light from the source **408** is split off by a 2x2 fibre coupler **414** into a reference arm **416** and collimated with a spherical lens **418** to provide a collimated reference beam **420.** The remainder of the source light is directed into a sample arm **422** and collimated in the *y*-axis by a cylindrical lens **424** to provide a sample beam **426** incident on a beam splitter **428,** depicted here as a power beam splitter although a polarisation beam splitter may be used. The sample light transmitted through the beam splitter **428** is focused in the *y*-axis and collimated in the *x*-axis by action of a spherical lens **430** to illuminate a volume **404** of the object **402** having a highly elongated cross-sectional area **405.** For clarity the object **402** and the elongated illuminated area **405** are depicted in oblique view, with the short and long axes of the illuminated area parallel to the *y-* and x-axes respectively.

The dimensions of the illuminated area **405** are determined by the numerical aperture of the optical fibre **410** and the focal lengths of the spherical lens **430** and cylindrical lens **424.** For example the combination of an optical fibre **410** of numerical aperture 0.1, a cylindrical lens **424** of focal length 2 mm and a spherical lens **430** of focal length 50 mm will provide an illuminated area **405** of approximately 200 µm x 10 mm, corresponding to an aspect ratio of fifty. In certain embodiments one or more apertures are included in the optical train to create a sharp rectangular illumination profile rather than the Gaussian profile of a singlemode optical fibre.

While the elongated illumination profile is incident on the object **402** with a relatively low numerical aperture, light **432** is reflected or scattered from the illuminated volume **404** over a much larger cone of angles. The reflected/scattered spherical wavefronts are collimated by the lens **430** and reflected at the beam splitter **428** where they interfere with the reference beam **420.** The interference pattern **434** proceeds via a lens relay comprising a pair of spherical lenses **454A, 454B** and a linear aperture **456** to a spatial sampling element in the form of a cylindrical lenslet array **436,** where it is sampled in the far field in one axis, i.e. the short axis of the illuminated area **405,** based on the pitch of the lenslet array **436.** The lens relay **454A, 454B,** in combination with the lens **430,** forms an optical system for capturing and anisotropically transforming the light **432** reflected or scattered from the illuminated volume **404.**

The linear beamlets **438** produced by the cylindrical lenslet array **436** are projected onto a linear aperture array **440** for rejection of light from angles not corresponding to the illuminated volume **404,** e.g. multi-scattered light, to provide a well-defined image for analysis in a spectrometer **442,** for obtaining phase and amplitude information over a range of wavelengths.

As in the previous embodiments the spectrometer comprises lens relay elements **444, 446** and a wavelength dispersive element **448** shown here as a grating. In other embodiments the dispersive and optical power elements can be , e.g. in the form of a chirped grating where the variation in grating spacing can provide the optical power for focusing. The wavelength dispersed re-imaging of the aperture array plane onto a 2-D sensor array **450** now provides a Fourier plane interference for each of the different wavelengths, for analysis in a computer **452** using methods described for the Fourier plane sampling embodiments in US 2016/0345820 A1. The use of an etalon **412** to extend the coherence depth offers the opportunity for digital refocusing beyond the Nyquist range. When refocused, the light scattered/reflected from the elongated illuminated volume **404** can be processed into a volume acquired in a single shot, for registration with subsequent volumes acquired with relative motion between the object **402** and the illuminated area **405.** Preferably there is some overlap between adjacent volumes to facilitate the registration.

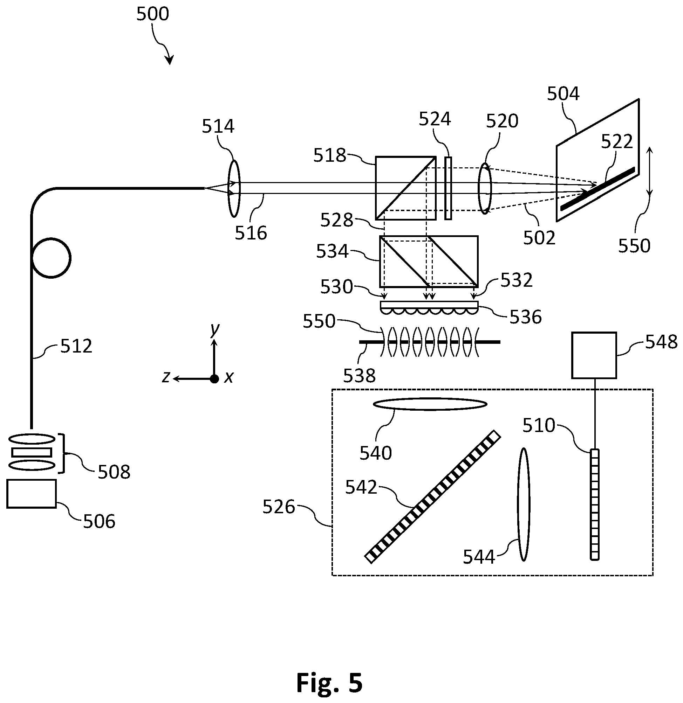

Figure 5 shows in schematic plan view an apparatus **500** for multi-wavelength wavefront sensing or analysis of light **502** reflected or scattered from an object **504,** according to another embodiment of the present invention. This apparatus does not require a reference beam or a spectrally coherent source and is in some ways similar to that depicted in Figure 4 but without a reference arm. This approach, in which an object **504** is illuminated by a partially incoherent light source **506** and information obtained in the Fourier field, i.e. the far field, has several applications including shear wave interferometry and speckle propagation wavefront sensing. The ability to sample the far field at multiple wavelengths is useful in increasing the speckle contrast, which requires some degree of spectral coherence, and for providing wavelength-dependent information that can be used for tomography or precise determination of a scattering point for metrology. The degree of spectral coherence can be enhanced with wavelength filtering.

The apparatus **500** of Figure 5 is described with respect to speckle tracking of the far field at two different propagation distances, an application that can be thought of as a multi-wavelength generalisation of a technique disclosed in S. Bonaque-Gonzalez *et al* 'Extremely high resolution ocular aberrometry up to 2.4 million points', *Investigative Ophthalmology & Visual Science* **60**, 603 (July 2019). Light from a broadband source such as a SLED **506** has its wavelength range restricted with a band-pass filter **508** to ensure that the spectra do not overlap on a 2-D sensor array **510,** then is coupled into an optical fibre **512** and collimated in the y-axis with a cylindrical lens **514** to provide a multi-wavelength probe beam **516** diverging in the x-axis. At least a portion of the probe beam **516** is transmitted by a polarisation beam splitter **518,** then focused in the *y*-axis and collimated in the *x*-axis by a spherical lens **520** to illuminate an elongated area **522** of an object **504.** It should be noted however that for this speckle tracking application the shape of the illuminated area **522** may be unimportant if the wavefront is not being used to construct an image with near-isotropic resolution, and the cylindrical lens **514** may for example be replaced by a spherical lens to provide a more symmetrical illuminated area. The double pass through a quarter waveplate **524** allows the back-scattered or reflected light **502** to be redirected by the polarisation beam splitter **518** towards a spectrometer **526.** In this embodiment the captured optical field **528** reflected or scattered from the illuminated area **522,** which is in the Fourier plane due to the collimation by the lens **520,** is split into a direct path **530** and a delayed path **532** by a splitting element **534.** Light in both paths **530, 532** is sampled in the Fourier plane in one axis, i.e. the short axis of the illuminated area **522,** by a spatial sampling element in the form of a cylindrical lenslet array **536** and the resulting linear beamlets **550** passed through a linear aperture array **538** before being measured simultaneously over a range of wavelengths by a spectrometer **526** with components **540, 542, 544** and **510** identical to those employed in the apparatus **400** of Figure 4. In the illustrated embodiment the splitting element **534** is in the form of a compound beam splitting prism, which could be extended to provide additional paths with increasingly large delays with respect to the direct path **530.** In alternative embodiments the splitting element **534** may be a shearing interferometer or other self-interferometric system. The direct and delayed path measurements for each wavelength are processed by a computer **548** equipped with suitable machine-readable code, with the simultaneous capture of the data ensuring that phase relationships between light in the two paths are preserved. As in Figure 4 extended portions of the object **504** can be acquired by scanning the elongated illuminated area **522** relative to the object, e.g. in a direction **550** substantially parallel to the short axis of the illuminated area **522.** The computer **548** may for example process the direct and delayed path measurements to provide a measure of an optical wavefront formed by the light **502** scattered or reflected from the illuminated area **522.** The apparatus **500** may for example be configured as an aberrometer for illuminating and capturing light reflected or scattered from the fovea of an eye, for providing a measure of the power or aberrations of the eye.

The apparatus **500** shown in Figure 5 includes a spherical lens **520** for focusing the illumination onto the object **504** and for collecting a portion of the reflected or scattered light **502.** Comparison with Figure 2A shows that this lens is generally not required if the apparatus is being used to inspect the retina **202** or fovea of an eye **204,** unless the eye's optical power elements **248, 250** are unable to provide the requisite imaging due to aberration or non-emmetropia. Similarly, the spherical lens **430** in the apparatus **400** depicted in Figure 4 may not be required if the apparatus **400** is being used to inspect a retina.

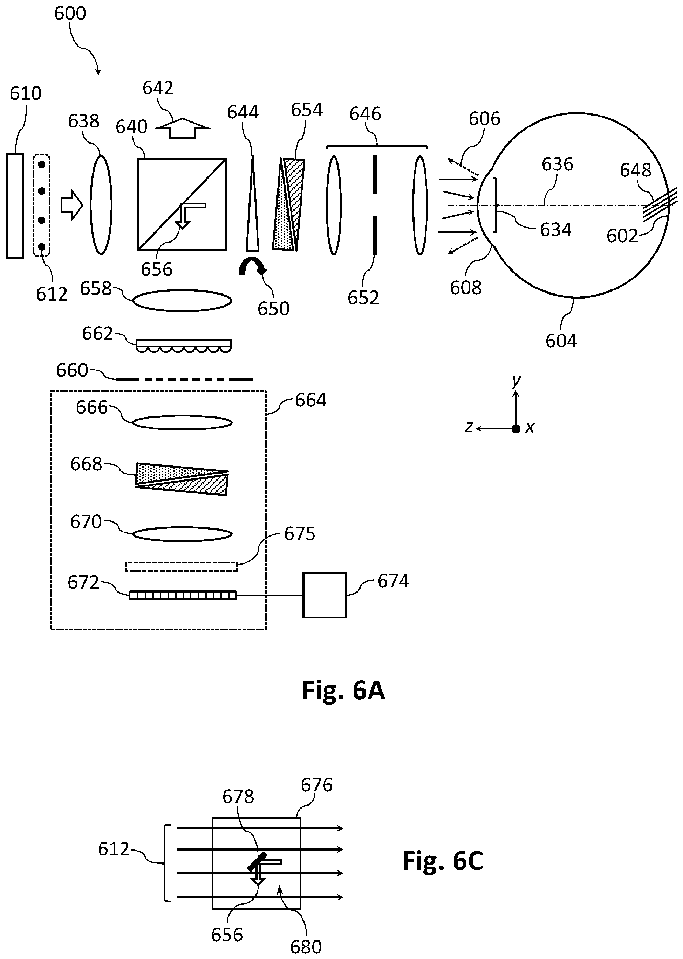

Figure 6A depicts in schematic plan view an apparatus **600** for *in-vivo* wide field hyperspectral imaging of the retina **602** of an eye **604,** according to another embodiment of the present invention. The apparatus **600** is configured for linear confocal imaging of the retina **602** with rejection of light **606** specularly reflected from the cornea **608.** The rejection of specular reflections, together with the ability to illuminate the retina only at required areas, provides improved signal-to-noise ratio compared to conventional hyperpectral imaging systems.

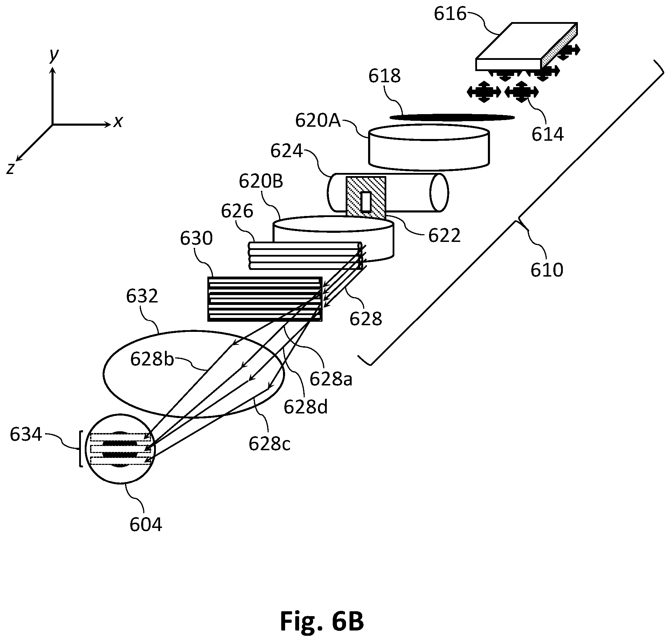

An important component of the apparatus **600** is an illuminator **610** configured to produce an array of thin illumination stripes **612** for projection onto the retina **602.** In the plan view of Figure 6A these illumination stripes **612** extend into the page (*x*-axis). The bandwidth of the illuminator **610** may be chosen to provide a wavelength range covering selected spectral reflection or absorption features of the retina, or more generally of an object to be analysed. A construction of a suitable illuminator **610** according to certain embodiments is described with reference to the schematic oblique view shown in Figure 6B. An array of blue LEDs **614** illuminates a thin plate **616** of Ce-YAG or other phosphor of a desired spectral range, to produce a stripe **618** of broadband light that is spatially incoherent to a large extent, at least in the long axis, defined here as the x-axis. The use of a low spatial coherence source is advantageous for safety in *in-vivo* ocular examination because the emitted light cannot be focused to a small high-intensity spot on the retina. Recycling of the LED power may be provided by a high reflectance coating on selected surfaces of the plate **616.** The plate may also be surrounded by an intermediate refractive index material such as glass to reduce the numerical aperture of the emitted beam **618** in the *y*-axis compared to the case with an air-clad plate, with the structure optionally designed to provide singlemode waveguiding in the *y*-axis. In this example embodiment we will consider a Ce-YAG plate **616** of approximate dimensions 5x5x0.1 mm emitting a beam **618** of initial approximate dimensions 5 mm in the *x*-axis and 0.1 mm in the *y*-axis.

The emitted beam **618** is imaged in the *x*-axis by cylindrical lenses **620A, 620B** each having focal length 10 mm and configured in combination to provide no magnification, although they may be configured to provide magnification if desired. In certain embodiments the beam **618** is spatially filtered by one or more apertures **622** to provide an appropriate numerical aperture and to control regions where the light is projected onto the cornea. The beam **618** may also be filtered spectrally, e.g. with a band-pass filter, to limit the wavelength range. A cylindrical lens **624** of focal length 20 mm is positioned to collimate or magnify the beam **618** in the *y*-axis, and the collimated or magnified beam directed to an array of one-dimensional focusing elements **626** such as an array of cylindrical lenslets on a 200 µm pitch having focal length 1 mm. In a preferred embodiment the cylindrical lenses **620A, 620B** and **624** form a 4F/2F system with the cylindrical lenslet array **626** positioned at or near an image plane in the *y*-axis and at or near a Fourier plane in the *x*-axis. The lenslets **626** are oriented to focus in the *y*-axis, producing a plurality of beamlets **628** elongated in the x-axis. In the illustrated embodiment the beamlets proceed to a structured plate **630** configured with a number of facets for steering individual beamlets in selected directions, with the plate preferably positioned at a focal plane of the cylindrical lenslet array **626** so that the beamlets **628** can be redirected to enter the pupil from different directions without changing their position on the retina. In the illustrated example the structured plate **630** is configured such that the peripheral beamlets **628a, 628d** propagate without deviation while the central beamlets **628b, 628c** are redirected up and down respectively. One or more optical power elements in the source-to-eye path, described below with reference to Figure 6A but represented here by a single spherical lens **632,** project the beamlets **628** onto a plurality of locations **634** on the eye **604.** The focusing action of the eye then maps the light from these locations **634** onto elongated locations on the retina corresponding to the position of the beamlets within the beamlet array **628.**

Operation of the hyperspectral imaging apparatus **600** will now be described. Returning to Figure 6A, the illuminator **610** emits an array of thin illumination stripes **612** that correspond to the elongated beamlets **628** formed by the cylindrical lenslet array **626** as shown in Figure 6B. Each illumination stripe can be considered as a separate spatially incoherent source, with its own angular trajectory in the *x*- and *y*-axes as determined by the structured plate **630.** In preferred embodiments this plate is designed to steer each illumination stripe such that specular reflections **606** from the cornea **608** occur at sufficiently large angles to the ocular axis **636,** e.g. greater than 12 degrees, for them not to be captured. Capture of corneal reflections is also suppressed by virtue of the fact that the elongated beamlets are focused at the retina, not at the cornea. For non-ocular samples, or if corneal reflections are of minor concern, the structured plate **630** may be omitted. The illumination stripes **612** are collimated with a spherical lens **638** then transmitted through a polarising or non-polarising beam splitting cube **640,** with reflected power **642** optionally used to provide a reference beam for coherence-resolved imaging or OCT. The transmitted beamlets proceed to an optional angular deflection element **644,** shown here as transmissive although it may be reflective, configured to deflect the beamlets in the *y-*axis with a θₓ angular deflection **650.** The beamlets are relayed to a plurality of locations **634** on the cornea **608** by a focal plane relay **646,** with the optical power elements of the eye **604** creating an image **648** on the retina **602.** For clarity the on-retina image **648** of the illumination stripes **612** is shown in oblique view as a series of parallel lines. Preferably the apparatus **600** is positioned relative to the eye **604** such that the illuminating light is able to pass through an undilated pupil (not shown), unlike in conventional hyperspectral imaging systems. Although the illumination may be clipped by a constricted pupil in the case of misalignment, this will not affect the location of the image **648** on the retina **602.** In general a confocal system using visible light and with good resolution on the eye only requires a pupil size of 1 mm. The position of the image **648** on the retina **602** can be tuned by angular adjustment **650** of the deflection element **644.**

In certain embodiments the focal plane relay **646** has focal adjustment capability for optimising formation of the on-retina image **648** and may include an aperture **652** for rejection of specularly reflected light **606.** This is in addition to the spatial filtering offered by the confocal nature of the imaging system, described below. The optical train may also include a dispersive element **654** for dispersing light in each illumination line **612** over a larger area of the retina **602** for increased patient comfort, and potentially for facilitating registration of subsequent frames of an acquisition. For these purposes the dispersion direction should be at an angle to the long axis of the illumination lines, more preferably substantially perpendicular to the long axis. In the illustrated embodiment the dispersive element **654** comprises a pair of wedges of materials of different wavelength dispersive properties, with a combination of PMMA and fused silica for example providing a cost-effective dispersive element with low nonlinearity of dispersion. Any dispersion imposed on the beamlets on the outward path will be reversed on the return path, so as not to interfere with the passage of the return light through the confocal linear aperture array **660** described below.

Return light **656** scattered, reflected or fluoresced from the retina **602** within the acceptance angle of the relay optics **646** is reflected by the beam splitter **640** and imaged by a spherical lens **658** onto a linear aperture array **660.** A cylindrical lenslet array **662** may be included to convert the numerical aperture of each line, i.e. reduce the spot size in the short axis, for optimal capture of the reflected light through the slits of the aperture array **660.** The aperture array is an important component of the hyperspectral imaging apparatus **600,** since it creates a linear confocal system that rejects light scattered or reflected from points away from the focal plane, which in this embodiment is positioned at or near the retina **602.** The return light passed by the aperture array **660** proceeds to a spectrometer **664** for simultaneous detection, over a range of wavelengths, of the return light. The spectrometer comprises a collimating lens **666,** a dispersive element **668** and a focusing lens **670** for focusing and directing the dispersed stripes of scattered/reflected/fluoresced light onto a 2-D focal plane array **672** for read out and processing in a computer **674** equipped with suitable machine-readable code. The dispersive element **668** is oriented to disperse the light stripes in the direction substantially perpendicular to their long dimension, and in the illustrated embodiment comprises a wedged pair. In certain embodiments the dispersive element **668** is birefringent, e.g. by choosing crystalline quartz for one of the wedges and an isotropic material for the other, enabling polarisation diverse detection. In another embodiment polarisation diverse detection is provided by the addition of a walk-off plate **675** in the spectrometer **664.**

As shown in Figure 6C, in an alternative embodiment the beam splitting cube **640** is replaced by a spatially selective directional coupling element in the form of an aperture reflection beam splitter **676** having a reflective area **678** and a transmissive area **680,** positioned such that a return signal **656** reflects off the reflective area **678** while allowing the illumination, which could be an array of illumination stripes **612** generated by the illuminator **610** shown in Figure 6B or some other suitable illuminator, to pass through the transmissive area **680.** This embodiment provides greater selectivity of the return signal, rejecting high numerical aperture light arising for example from specular reflections or aberrations in the eye **604.**

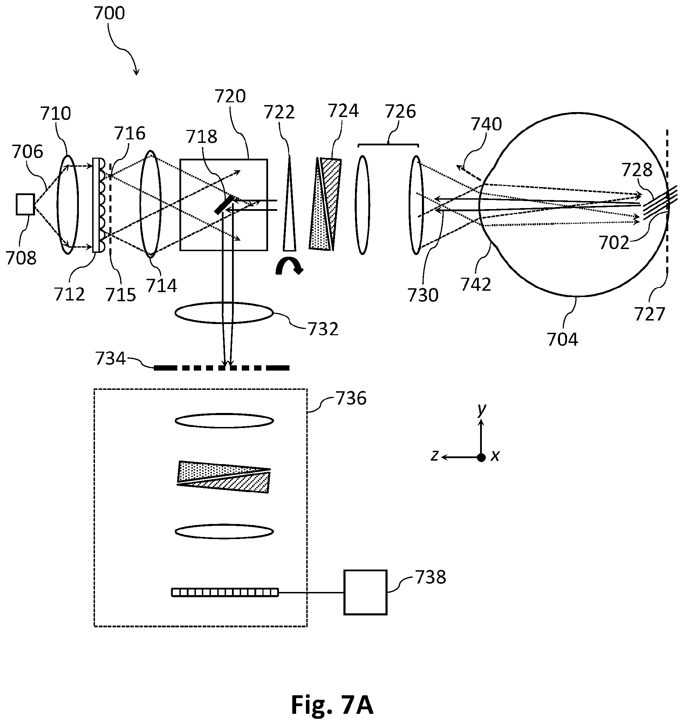

Figure 7A depicts in schematic plan view an apparatus **700** for *in-vivo* wide field hyperspectral imaging of the retina **702** of an eye **704,** according to another embodiment of the present invention. Light **706** from a broadband optical source **708** such as a SLED is collimated with a spherical lens **710** then passed through a lenslet array **712,** which in a preferred embodiment is an array of cylindrical lenslets to produce, at a focal plane **715** of the lenslet array **712**, a structured illumination field **716** comprising an array of beamlets elongated in the *x*-axis. An optical power element in the form of a spherical lens **714** positioned in the far field of the structured illumination field **716** collimates the beamlets, which then proceed towards the eye **704** via an aperture reflection beam splitter **720** similar to that depicted in Figure 6C, generally with some loss of intensity at the reflective area **718,** followed by an optional angular deflection element **722,** dispersive element **724** and lens relay **726,** before being imaged to a focal plane **727** at or near the retina **702** by the optical power of the eye. In general the image **728** on the retina, shown in oblique view, will comprise a set of possibly overlapping areas corresponding to the wavelength-dispersed beamlets of the structured illumination field **716** if the dispersive element **724** is present, or a set of multi-wavelength lines if the dispersive element is absent.

Light **730** reflected, scattered or fluoresced from the illuminated lines or areas of the image **728** within the acceptance angle of the reflective area **718** of the aperture reflection beam splitter **720** is focused by a lens **732** and spatially filtered with a linear aperture array **734** then analysed spectrally by a spectrometer **736** and a computer **738** as described previously. Reflections **740** from the cornea **742** are substantially excluded from the spectrometer **736** by the fact that the numerical apertures corresponding to the transmissive and reflective portions of the aperture reflection beam splitter **720** converge only at the focal plane **727** positioned at or near the retina **702.** This focal plane **727** is conjugate to the focal plane **715** of the lenslet array **712** and the linear aperture array **734.** A cylindrical lenslet array may be provided in front of the linear aperture array **734** to improve the collection efficiency by allowing a transformation of the numerical aperture or spot size.

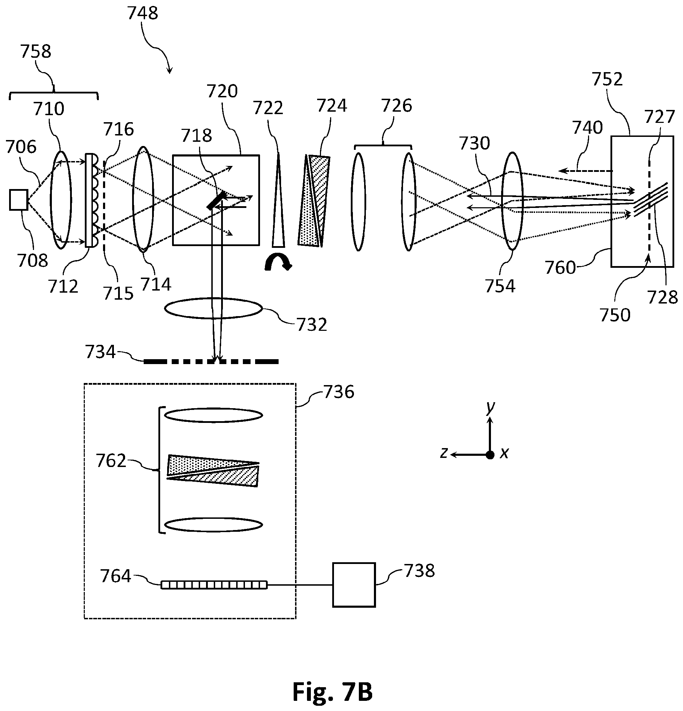

Figure 7B depicts in schematic plan view an apparatus **748** for hyperspectral confocal imaging of a sub-surface region **750** of an object **752** that may for example be the cornea or retina of an eye or a non-ocular sample. Sub-surface regions that may be of interest include the endothelium of the cornea and the retinal nerve fibre layer. The apparatus **748** is a variation of the apparatus **700** shown in Figure 7A, modified with the inclusion of an objective lens **754** for projecting the illumination onto a target sub-surface region **750** and for collecting light **730** reflected, scattered or fluoresced from the imaged illumination field **728.** The apparatus **748** may be useful for food inspection, e.g. for analysis of layers of fruit a short distance below the surface, while suppressing reflections from the surface.

A broadband optical source **708,** lens **710** and cylindrical lenslet array **712** in combination form an illuminator **758** for forming, at a focal plane **715** of the lenslet array **712,** a structured illumination field **716** comprising an array of beamlets elongated in the *x*-axis. An optical power element in the form of a spherical lens **714** positioned in the far field of the structured illumination field **716** collimates the beamlets, which then proceed towards the object **752** via an aperture reflection beam splitter **720** followed by an optional angular deflection element **722,** dispersive element **724** and lens relay **726,** before being imaged by the objective **754** to a focal plane **727** at the target sub-surface region **750.** The axial depth of the imaged illumination field **728** within the object **752** will depend, for a given distance between the apparatus **748** and the object, on the details of the optics in the illumination train. As in the previous embodiment shown in Figure 7A the imaged illumination field **728,** shown here in oblique view. may comprise a set of possibly overlapping areas or a set of multi-wavelength lines, depending on whether a dispersive element **724** is present.

The spatially selective directional coupling element **720** with its reflective portion **718** is positioned so as to direct at least a portion of the structured illumination field **716** towards the object **752** and to direct at least a portion of the reflected, scattered or fluoresced light **730** towards a spatially structured confocal aperture in the form of a linear aperture array **734.** Light passed by the linear aperture array proceeds to a spectrometer **736** comprising a dispersive optical relay **762** that projects a wavelength-dispersed image of the linear aperture array onto a 2-D sensor array **764** for read out and processing in a computer **738** equipped with suitable machine-readable code. Similar to the embodiment shown in Figure 7A, reflections **740** from surfaces in front of or behind the target sub-surface region **750,** in particular from the front surface **760** of the object **752,** are suppressed by the fact that the numerical apertures corresponding to the transmissive and reflective portions of the aperture reflection beam splitter **720** converge only at the focal plane **727** positioned at the target sub-surface region **750.** As in Figure 7A the focal plane **727** is conjugate to the focal plane **715** of the lenslet array **712** and the linear aperture array **734,** and a cylindrical lenslet array may be provided in front of the linear aperture array **734** to improve the collection efficiency.

In alternative embodiments the apparatus **700, 748** depicted in Figures 7A and 7B include a 2-D array of spherical lenslets instead of the cylindrical lenslet array **712,** in which case the structured illumination field **716** will comprise a 2-D array of beamlets. In these embodiments the imaging side of the apparatus includes a 2-D aperture array in place of the linear aperture array **734**, with the orientation of the 2-D aperture array with respect to the dispersion axis of the dispersive optical relay **762** preferably chosen to ensure that each dispersed beamlet is mapped onto a unique set of pixels of the 2-D sensor array **764.**

The various apparatus described above have been depicted in reflective configurations, e.g. for *in-vivo* retinal imaging. However it will be appreciated that in general the inventive principles could also be applied to transmissive configurations where light scattered or fluoresced from a partially transmissive sample is captured.

Although the invention has been described with reference to specific examples, it will be appreciated by those skilled in the art that the invention may be embodied in many other forms.

## Claims

1. An apparatus for wavefront sensing comprising:
an illumination system comprising an optical source for illuminating, with a multi-wavelength optical beam, an area of an object;
an optical system for capturing an optical wavefront comprising light scattered or reflected from the illuminated area;
an element for splitting the captured light into two or more paths having different optical delays;
a spatial sampling element for simultaneously sampling, in one dimension, the captured light in the two or more paths; and
a measurement system comprising a two-dimensional sensor array for simultaneous measurement, over a range of wavelengths, of the light in the two or more paths.

2. The apparatus according to claim 1, comprising a computer for analysing the measurement of the light in the two or more paths to provide a measure of said optical wavefront.

3. The apparatus according to claim 1 or claim 2, wherein said apparatus is configured to illuminate the fovea of an eye and capture light reflected or scattered from the fovea, for providing a measure of the power or aberrations of the eye.

4. A hyperspectral imaging apparatus comprising:
an illuminator for producing a plurality of elongated beams having a wavelength range covering selected spectral reflection or absorption features of an object;
an optical system for projecting said plurality of elongated beams onto a corresponding plurality of areas on said object and for capturing light reflected, scattered or fluoresced from said areas;
a linear aperture array for confocal gating of the captured light reflected, scattered or fluoresced from said areas; and
a spectrometer comprising a two-dimensional sensor array for simultaneous detection, over a range of wavelengths, of the captured light reflected, scattered or fluoresced from said areas.

5. The apparatus according to claim 4, wherein said apparatus is configured for hyperspectral imaging of an object comprising the retina of an eye.

6. The apparatus according to claim 5, wherein said optical system is configured to project said plurality of elongated beams towards the eye at angles selected to suppress the capture of specular reflections of said elongated beams from the cornea of the eye.

7. The apparatus according to claim 5 or claim 6, wherein said optical system comprises a focal plane relay including an aperture for suppressing the capture of specular reflections of said elongated beams from the cornea of the eye and/or a dispersive element for angularly dispersing said plurality of elongated beams, so as to reduce the intensity of the elongated beams on said retina.

8. The apparatus according to any one of claims 4 to 7, comprising a cylindrical lenslet array for focusing the captured light onto the apertures of said linear aperture array and/or a birefringent element for polarisation-sensitive detection of the captured light.

9. The apparatus according to any one of claims 4 to 8, comprising a beam splitter for directing said elongated beams towards the object and for directing the captured light towards said spectrometer, preferably said beam splitter is configured to provide a reference beam for coherence-resolved detection of the captured light.

10. The apparatus according to any one of claims 4 to 9, comprising a deflection element for moving the areas on said object in a direction substantially perpendicular to the long axes of said areas, for imaging larger areas of said object.

11. The apparatus according to any one of claims 4 to 10, wherein said illuminator comprises: an optical source for emitting an elongated stripe of light; and an array of one-dimensional focusing elements for producing said plurality of elongated beams from said elongated stripe of light, preferably said illuminator comprises an aperture for adjusting the spatial coherence of the light emitted from said optical source and/or a beam redirection element for steering individual beams of said plurality of elongated beams in selected directions, more preferably said beam redirection element is positioned at a focal plane of said array of one-dimensional focusing elements.

12. The apparatus according to any claim 11, wherein said optical source is substantially spatially incoherent.

13. An apparatus for hyperspectral confocal imaging of a sub-surface region of an object with enhanced rejection of surface reflected light, said apparatus comprising:
an illuminator for forming a spatially structured illumination field for projection onto a sub-surface region of an object;
an optical power element positioned in the far field of the structured illumination field;
a spatially structured confocal aperture configured to accept light reflected, scattered or fluoresced from said sub-surface region of said object;
a spectrometer comprising a dispersive optical relay for projecting a wavelength dispersed image of the structured confocal aperture onto a two-dimensional sensor array; and
a spatially selective directional coupling element for directing at least a portion of the structured illumination field towards said object and for directing at least a portion of the reflected, scattered or fluoresced light towards said spatially structured confocal aperture, such that the capture of light reflected from a front surface of said object is suppressed.

14. The apparatus according to claim 13, wherein said apparatus is configured for hyperspectral confocal imaging of a sub-surface region of an object comprising one among of the retina of an eye, and the cornea of an eye.

15. A method for wavefront sensing, said method comprising the steps of:
illuminating, with a multi-wavelength optical beam, an area of an object;
capturing an optical wavefront comprising light scattered or reflected from the illuminated area;
splitting the captured light into two or more paths having different optical delays;
simultaneously sampling, in one dimension, the captured light in the two or more paths; and
simultaneously measuring over a range of wavelengths, with a measurement system comprising a two-dimensional sensor array, the light in the two or more paths.

16. A method for hyperspectral imaging, said method comprising the steps of:
producing a plurality of elongated beams having a wavelength range covering selected spectral reflection or absorption features of an object;
projecting said plurality of elongated beams onto a corresponding plurality of areas on said object;
capturing light reflected, scattered or fluoresced from said areas;
confocally gating the captured light reflected, scattered or fluoresced from said areas; and
simultaneously detecting over a range of wavelengths, with a spectrometer comprising a two-dimensional sensor array, the captured light reflected, scattered or fluoresced from said areas.

17. A method for hyperspectral confocal imaging of a sub-surface region of an object with enhanced rejection of surface reflected light, said method comprising the steps of:
forming a spatially structured illumination field;
projecting, with a system of optics comprising an optical power element positioned in the far field of the structured illumination field, light in said structured illumination field onto said sub-surface region of said object;
accepting, with a spatially structured confocal aperture, light reflected, scattered or fluoresced from said sub-surface region of said object;
projecting a wavelength dispersed image of the confocally accepted light onto a two-dimensional sensor array; and
directing, with a spatially selective directional coupling element, at least a portion of the structured illumination field towards said object and directing at least a portion of the reflected, scattered or fluoresced light towards said spatially structured confocal aperture, such that the capture of light reflected from a front surface of said object is suppressed.
